# EUROPEAN PATENT SPECIFICATION

(11) **EP 0 476 573 B1**
(45) Date of publication and mention of the grant of the patent: **24.04.1996**
(21) Application number: 91115706.3
(22) Date of filing: 16.09.1991
(51) Int. Cl.: C07D 237/18, A01N 43/58

(54) **2-tButyl-4-chloro-5-(4-ibutylmethylcyclohexyl)-methylthio-3(H)-pyridazinone having acaricidal and insecticidal activity**
2-tButyl-4-Chlor-5-(4-iButoxymethylcyclohexyl)-methylthio-3(H)-Pyridazinon mit insektizider und akarizider Aktivität
2-tButyl-4-chloro-5-(4-ibutylmethylcyclohexyl)-methylthio-3(H)-pyridazinone ayant une activité insecticide et acaricide

(30) Priority: 17.09.1990 IT 2149690
(43) Date of publication of application: 25.03.1992
(73) Proprietor: MINISTERO DELL' UNIVERSITA' E DELLA RICERCA SCIENTIFICA E TECNOLOGICA, I-00196 Roma (IT)
(72) Inventor: Bettarini, Franco, Dr., I-28100 Novara (IT); Capuzzi, Luigi, Dr., I-28100 Novara (IT); Massimini, Sergio, Dr., I-20021 Bollate,Milan (IT); Castoro, Paolo, I-13100 Vercelli (IT); Caprioli, Vincenzo, Dr., I-27028 San Martino Siccomario,Pavia (IT)
(74) Representative: Weinhold, Peter, Dr.

(56) References cited:
- EP-A- 0 344 684
- EP-A- 0 377 892
- DE-A- 3 733 220

## Description

The present invention relates to a new derivative of 3(2H)-pyridazinone, acaricidal and insecticidal compostions containing it and the use thereof for controlling acari, insects, ticks, etc.

In particular, the invention relates to a new substituted pyridazinone exhibiting an increased efficacy in controlling particularly acari and insects which are noxious in the agrarian, civil and zootechnical fields.

3 (2H) -Pyridazinones having fungicidal, insecticidal, acaricidal and/or nematocidal activity are described, e.g., in EP-A-88384, 134439, 183212, 199281, 232825, 283271, 302346, 320733 and in DE-A-3733220.

The mentioned DE-A describes compounds of general formula (A): 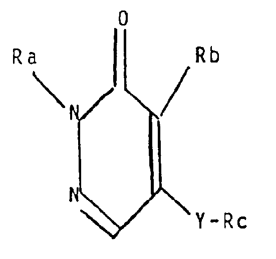 wherein Ra represents H or a C₁-C₆ alkyl group (optionally substituted with halogen or CH), Rb represents H, alkyl or halogen, Y represents 0 or S, and Rc is an optionally substituted C₁-C₂₀ alkyl group. Among the possible substituents of the C₁-C₂₀ alkyl group there are mentioned C₃-C₈ cycloalkyl groups, optionally substituted with OH or C₁-C₄ alkyl- carbonyloxy groups.

EP-A-0 377 892 discloses substituted compounds of general formula 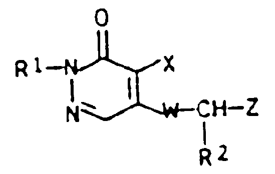 wherein R¹ represents a C₁-C₈ alkyl group, R² represents hydrogen or a C₁-C₄ group, X represents halogen, W represents oxygen or sulfur and Z is, for example, a Cₛor C₆ cycloalkyl group, optionally substituted e.g. by halogen, C₁-C₁₀ alkyl groups, C₁-C_{S} alkoxy groups or C₂-C_{S} alkoxyalkyl groups.

The present invention provides a pyridazone derivative, 2-tert-butyl-4-chloro-5-(4-isobutoxymethylcyclohexyl)-methylthio-3(2H)-pyridazinone (compound I), having higher acaricidal or insecticidal activity than the compounds of the prior art. Compound I is endowed with a high acaricidal and insecticidal activity towards acari and noxious insects in the agrarian, civil and zootechnical fields; in particular it exerts its action against important species of tetranids, hemiptera, lepidopters, coleopters, dipterans, blattodeas and ixodids

Compound (I) may be prepared, e.g., by reacting 2-tert-butyl-4-chloro-5-mercapto-3(2H)-pyridazinone and (4-iso- butoxymethylcyclohexyl)methyl bromide.

The reaction is preferably conducted in an inert organic solvent (such as benzene, toluene, acetone, methylethylketone, acetonitrile, dioxane, N,N-dimethylformamide, dimethylsulphoxide etc.) and in the presence of an inorganic base (such as sodium hydride, potassium hydroxide, calcium hydroxide, sodium carbonate, potassium carbonate etc.) or in the presence of an organic base (such as triethylamine, pyridine etc.). The reaction temperature usually ranges from room temperature to the boiling temperature of the solvent employed in the reaction.

Compounds such as 2-tert-butyl-4-chloro-5-mercapto-3(2H)-pyridazinone are described in the literature and may be prepared according to the procedure described, e.g., in "Advances in Heterocyclic Chemistry" vol. 9, pages 235-236 and 249-258, Ed. A.R. Katritzky, A.J. Boultin, Academic press, New York and London 1968, and the literature referred to therein.

(4-Isobutoxymethyl-cyclohexyl)methyl bromide can be prepared according to usual methods of organic chemistry.

Compound (I) can be in the form of mixtures of isomers, the separation of which can be carried out by using known chemical techniques such as column chromatography or thin layer chromatography

The isolation and use of each individual isomer, as well as the direct use of the mixtures obtainable from the preparation of compound (I) and the utilization of the mixtures resulting from an incomplete separation of the isomers are all within the scope of the present invention.

As mentioned above, compound (I) is endowed with a high insecticidal and, in particular, acaricidal activity which is exerted, in general, at all stages of the life cycle of acari and insects (larvae, adults and eggs); furthermore they possess an excellent residual activity.

Thanks to their favourable properties, the compound (I) is suited for use in the protection of both agrarian and horticultural cultures, environments with human beings, as well as domestic animals and cattle, from acari and noxious insects.

For their use in practice, both in agriculture and in other sectors, it is advantageous to employ the compound of the invention in the form of suitable compositions.

These compositions generally contain, besides compound (I) as active ingredient, solid inert carriers (e.g. kaolin, silica, talc, attapulgite, bentonite, diatomaceous earth, etc.) or inert liquid carriers (e.g. organic solvents, vegetable or mineral oils, water, and mixtures thereof) and optionally other additives which are usually utilized in the formulative sector, such as surfactants, suspending agents, dispersants and wetting agents.

For particular applicative requirements or in order to extend the compositions' range of activity it is possible to add other active ingredients, such as for example other insecticides and/or acaricides, herbicides, fungicides and/or fertilizers thereto.

The application doses vary as a function of various factors, such as type and degree of infestation, type of composition utilized and climatic and environmental factors.

For practical use in agriculture, doses of compound (I) in the range of from 5 g to 5 kg per hectare usually provide a satisfactory protection.

The following examples are given to further illustrate the present invention.

### EXAMPLE 1

Synthesis of 2-tert-butyl-4-chloro-5-(4-isobutoxymethyl-cyclohexyl)methylthio-3(2H)-pyridazinone

The above compound (cis/trans 13/87 mixture) was prepared as follows: To a suspension of 0.22 g of potassium carbonate in 5 ml of dimethylformamide 0.4 g of (4-isobutoxymethyl-cyclohexyl)methyl bromide and 0.35 g of 2-tert-butyl-4-chloro-5-mercapto-3 (2H) -pyridazinone were added. The resulting mixture was stirred for 16 hours at room temperature, then it was diluted with ether and washed with diluted HCI and brine. After drying the solvent was evaporated and the resulting crude product was subjected to silica gel chromatography with hexane/ethyl acetate 9/1 as eluent.
¹ H-NMR (60 MHz, CCI₄): 8 0.6-2.3 (m, 11 H); 0.85 (d, 6H); 1.55 (s, 9H); 2.85 (m, 2H); 2.9-3.3 (m, 4H); 7.4 (s, 1 H)

Compound (I) was tested under the conditions described in the following example.

### EXAMPLE 2

Determination of the acaricidal and insecticidal activity

### a) Acaricidal activity against Tetranychus urticae (TU.; Acari).

### Adults

Small discs obtained from bean leaves were infested with adult acari and then sprayed with an aqueous acetone solution (acetone: 10% by volume) of the test compound. The percentage of deaths was determined 48 hours after the treatment in comparison with the percentage of deaths of acari which infested discs sprayed only with the aqueous acetone.

### Eggs

Small discs obtained from bean leaves were infested with acari eggs and then treated by spraying an aqueous acetone solution of the test compound thereon. The percentage of non-hatched eggs was evaluated 7 days after the treatment in comparison with the percentage of eggs which had been treated only with the liquid carrier.

### b) Insecticidal activity against adults of Macrosiphum euphorbiae (M.E ; aphides)

Potatoe plants cultivated in pots were infested with aphide adult females and, after a few hours, were sprayed with an aqueous acetone suspension (10% by volume of acetone) of the test compound.

The mortality percentage of the aphides was determined 24 hours after the treatment in comparison with the mortality percentage of aphides which infested plants treated with the liquid carrier only.

The results of the above tests are reported in Table 1; said results are expressed as mortality percentage of the acari and insects treated with the test compounds at the indicated doses.

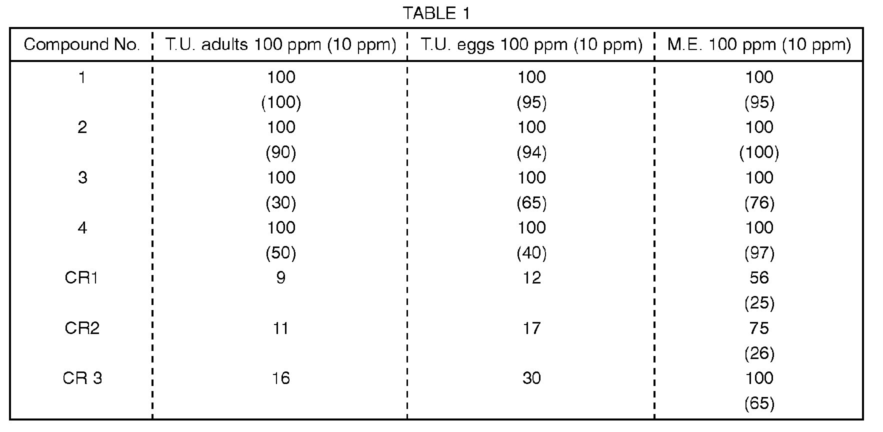

## Claims (Claims for the following Contracting State(s) : AT, BE, CH, DE, FR, GB, LI, NL)

1. 2-tert-butyl-4-chloro-5-(4-isobutoxymethylcyclohexyl)methylthio-3(2H)-pyridazinone.

2. Composition for controlling acari and/or noxious insect infestations, comprising the compound of claim 1, one or more solid or liquid vehicles, and, optionally, other usual additives and/or other active substances and/or fertilizers.

3. Use of the compound of claim 1 as an insecticide and/or acaricide.

## Claims (Claims for the following Contracting State(s) : ES)

1. Composition for controlling acari and/or noxious insect infestations, comprising 2-tert-butyl-4-chloro-5-(4-isobu- toxymethylcyclohexyl)-methylthio-3(2H)pyridazinone, one or more solid or liquid vehicles, and, optionally other usual additives and/or other active substances and/or fertilizers.

## Patentansprüche (Patentansprüche für folgende(n) Vertragsstaat(en) : AT, BE, CH, DE, FR, GB, LI, NL)

1. 2-tert-Butyl-4-chlor-5-(4-isobutoxymethylcycohexyl)methylthio-3(2H)-pyridazinon.

2. Zusammensetzung zur Bekämpfung von Befall mit Milben und/oder schädlichen Insekten, die die Verbindung nach Anspruch 1, einen oder mehrere feste oder flüssige Träger und gegebenenfalls andere gebräuchliche Additive und/oder andere aktive Substanzen und/oder Düngemittel umfaßt.

3. Verwendung der Verbindung nach Anspruch 1 als Insektizid und/oder Akarizid.

## Patentansprüche (Patentansprüche für folgende(n) Vertragsstaat(en) : ES)

1. Zusammensetzung zur Bekämpfung von Befall mit Milben und/oder schädlichen Insekten, die 2-tert-Butyl-4-chlor-5-(4-isobutoxymethylcyclohexyl)-methylthio-3(2H)-pyridazinon, eine oder mehrere feste oder flüssige Träger und gegebenenfalls andere gebräuchliche Additive und/oder andere aktive Substanzen und/oder Düngemittel umfaßt.

## Revendications (Revendications pour l'(les) Etat(s) contractant(s) suivant(s) : suivants : AT, BE, CH, DE, FR, GB, LI, NL)

1. 2-Tertiobutyl-4-chloro-5-[(4-isobutoxyméthyl-cyclohexyl)méthylthio]-3(2H)-pyridazinone.

2. Composition destinée à lutter contre les infestations d'acariens et/ou d'insectes nuisibles, contenant du composé conforme à la revendication 1, un ou plusieurs véhicules liquides ou solides et, le cas échéant, d'autres adjuvants habituels et/ou d'autres substances actives et/ou des agents fertilisants.

3. Emploi du composé conforme à la revendication 1, comme insecticide et/ou acaricide.

## Revendications (Revendications pour l'(les) Etat(s) contractant(s) suivant(s) : suivant : ES)

1. Composition destinée à lutter contre les infestations d'acariens et/ou d'insectes nuisibles, contenant de la 2-ter- tiobutyl-4-chloro-5-[(4-isobutoxyméthyl-cyclohexyl)-méthylthio]-3 (2H)-pyridazinone, un ou plusieurs véhicules liquides ou solides et, le cas échéant, d'autres adjuvants habituels et/ou d'autres substances actives et/ou des agents fertilisants.
